# EUROPÄISCHE PATENTANMELDUNG

(11) **EP 0 719 786 A2**
(43) Veröffentlichungstag der Anmeldung: **03.07.1996**
(21) Anmeldenummer: 95116019.1
(22) Anmeldetag: 11.10.1995
(51) Int. Cl.: C07H 15/04

(54) **Verfahren zur destillativen Aufarbeitung von fettalkoholischen Alkylpolyglycosid-Lösungen**

(30) Priorität: 03.12.1994 DE 4443089
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

Die Erfindung betrifft ein Verfahren zur Aufarbeitung von fettalkoholischen Alkylpolyglycosid-Lösungen, bei der die Leichtsieder, insbesondere Butanol und Wasser, destillativ abgetrennt werden und wobei der dabei mitgerissene Fettalkohol in einem angeschlossenen Rektifikationsteil aus den Leichtsiederbrüden entfernt wird.

## Beschreibung

Die Erfindung betrifft ein Verfahren zur Aufarbeitung von fettalkoholischen Alkylpolyglycosid-Lösungen, bei dem die Leichtsieder, insbesondere Butanol und Wasser, destillativ abgetrennt werden und wobei der dabei mitgerissene Fettalkohol in einem angeschlossenen Rektifikationsteil aus den Leichtsiederbrüden entfernt wird.

Alkylpolyglycoside sind ungiftige, leicht abbaubare oberflächenaktive Substanzen, die als Wasch- und Reinigungsmittel und als Emulgatoren und Dispergatoren verwendet werden.
Alkylpolyglycoside können einstufig oder zweistufig durch Glycosidierung und Umglycosidierung hergestellt werden. Sie enthalten im allgemeinen Alkylgruppen mit 8 bis 18 Kohlenstoffatomen, wobei Alkylgruppen mit 12 bis 16 Kohlenstoffatomen vorzugsweise vorhanden sind. Der mittlere Polymerisationsgrad liegt bei 1,05 bis 1,5, vorzugsweise bei 1,2 bis 1,5, ganz besonders bevorzugt bei 1,2 bis 1,4. Als einsetzbare Kohlenhydrate kommen Monosaccharide, wie Pentosen und Hexosen, Disaccharide, wie Saccharose und Maltose, und Polysaccharide, wie Stärke, in Betracht.

Ein einstufiges Herstellungsverfahren wird u. a. in der Patentanmeldung P 41 01 252.6 beschrieben.
Ein zweistufiges Verfahren wird in EP-A-0 306 652 angegeben, wobei zunächst durch Glycosidierung mit n-Butanol ein n-Butylglycosid und daraus durch Umglycosidierung mit einem langkettigen Alkohol das gewünschte langkettige Alkylpolyglycosid hergestellt wird.
Die Reaktion wird normalerweise mit einem großen Überschuß an Alkohol durchgeführt, so daß als Reaktionsprodukt ein Gemisch aus Alkylpolyglycosiden und Alkoholen vorliegt. Diese Alkohole beeinträchtigen die anwendungstechnischen Eigenschaften der Alkylpolyglycoside und müssen daher abgetrennt werden.

Bei dem zweistufigen Herstellverfahren verbleiben nach erfolgter Umglycosidierung noch geringe Mengen an kurzkettigen Alkoholen in der Reaktionslösung, vornehmlich C₄-Alkohol, der ebenfalls abgetrennt werden muß. Beide Reaktionen, Glycosiderung und Umglycosidierung, werden durch Säure katalysiert. Nach beendeter Reaktion wird mit Alkali- bzw. Erdalkalihydroxiden oder Alkoholaten neutralisiert. Die dabei zugeführten oder entstehenden sowie die bereits enthaltenen Leichtsieder, vornehmlich Wasser und kurzkettige Alkohole, werden wie der überschüssige Fettalkohol abdestilliert. Da die Siedepunkte der Alkohole, vornehmlich mit 8 oder mehr Kohlenstoffatomen, sehr hochliegen, und da sich bereits bei Temperaturen über 150 °C u.a. nicht umgesetzte Kohlenhydratreste unter unerwünschter Dunkelfärbung zersetzen, wird die destillative Abtrennung der Alkohole unter Feinvakuum durchgeführt.

In EP 0 092 876 werden Fettalkohole aus fettalkoholischen Alkylpolyglycosid-Lösungen mit Hilfe eines Dünnschichtverdampfers abdestilliert. In der Patentanmeldung DE 39 32 173 wird ein Verfahren angegeben, bei dem die Fettalkohole in zwei Stufen abgetrennt werden. In der ersten Stufe wird ein Fallfilmverdampfer mit einer Sumpftemperatur zwischen 100 und 220 °C und einem Druck von 1 bis 20 mbar eingesetzt. Beim nachgeschalteten Dünnschichtverdampfer liegt die Sumpftemperatur zwischen 120 und 250 °C und der Druck bei 0,1 bis 5 mbar.

Die vorgenannten Verfahren haben den Nachteil, daß die im Fettalkohol vorhandenen Leichtsieder unter den angegebenen Betriebsbedingungen ein großes Gasvolumen einnehmen und daher nur mit großem apparativen Aufwand entfernt werden können. Ein weiteres Problem ist, daß die Kondensationstemperaturen der Leichtsieder unterhalb der Erstarrungspunkte der Fettalkohole liegen und damit die Kondensation der Leichtsieder mit dem Erstarren der Fettalkohole einhergeht.

Die Aufgabe der Erfindung bestand daher in der Entwicklung eines Verfahrens zur destillativen Abtrennung der Leichtsieder, das bei vertretbaren Gesamtkosten den Leichtsiederanteil von etwa 2 % auf 0,1 % absenkt und der eigentlichen Fettalkoholabtrennung vorgeschaltet ist. Die Aufgabe wird erfindungsgemäß dadurch gelöst, daß ein der Fettalkoholabtrennung vorgeschalteter Fallfilmverdampfer mit nachfolgendem Rektifikationsteil eingesetzt wird.

Gegenstand der Erfindung ist mithin ein Verfahren zur Aufarbeitung von fettalkoholischen Alkylpolyglycosid-Lösungen, dadurch gekennzeichnet, daß die Leichtsieder destillativ abgetrennt werden, wobei die mitgerissenen Fettalkohole in einem Rektifikationsteil aus den Leichtsiederbrüden entfernt werden.

Die Leichtsieder werden bei 80 bis 170 °C, vorzugsweise bei 100 bis 150 °C und einem Druck von 10 bis 100 mbar, vorzugsweise 15 bis 50 mbar, abdestilliert.

Nachfolgend wird der Fettalkohol zum Beispiel mit Hilfe eines Dünnschichtverdampfers abgetrennt.

Statt eines Dünnschichtverdampfers kann aber auch ein Kurzwegverdampfer eingesetzt werden. Weiterhin sind auch Kombinationen dieser Verdampfertypen, eventuell auch mit Verwendung eines oder mehrerer Fallfilmverdampfer, möglich.

Das Verfahren hat den Vorteil, daß nach der Leichtsiederabtrennung mit verhältnismäßig geringem apparativen Aufwand ein gutes Vakuum erzeugt werden kann, so daß der Fettalkohol ohne größere thermische Belastung des Alkylpolyglycosids in ausreichendem Maß abgetrennt werden kann.

## Patentansprüche

1. Verfahren zur Aufarbeitung von fettalkoholischen AlkylpolyglycosidLösungen, dadurch gekennzeichnet, daß die Leichtsieder destillativ abgetrennt werden, wobei die mitgerissenen Fettalkohole in einem Rektifikationsteil aus den Leichtsiederbrüden entfernt werden.

2. Verfahren nach Anspruch 1, dadurch gekennzeichnet, daß die Abtrennung der Leichtsieder bei 100 bis 150 °C erfolgt.

3. Verfahren nach Anspruch 1, dadurch gekennzeichnet, daß die Abtrennung der Leichtsieder bei 15 bis 50 mbar erfolgt.

4. Verfahren nach Anspruch 1, dadurch gekennzeichnet, daß das Alkylpolyglycosid ein Alkylpolyglucosid ist.
